# EUROPEAN PATENT SPECIFICATION

(11) **EP 3 357 483 B1**
(45) Date of publication and mention of the grant of the patent: **27.01.2021**
(21) Application number: 16851844.7
(22) Date of filing: 30.09.2016
(51) Int. Cl.: A61K 8/35, A61K 8/19, A61K 8/36, A61Q 17/04, A61K 8/02

(54) **SUNSCREEN COSMETIC**
SONNENSCHUTZ-KOSMETIKUM
PRODUIT COSMÉTIQUE DE PROTECTION SOLAIRE

(30) Priority: 30.09.2015 JP 2015194610; 28.09.2016 JP 2016188954
(43) Date of publication of application: 08.08.2018
(73) Proprietor: Shiseido Company Ltd., Chuo-ku Tokyo 104-0061 (JP)
(72) Inventor: NAGAI, Kouichi, Yokohama-shi Kanagawa 224-8558 (JP); MATSUDA, Takashi, Yokohama-shi Kanagawa 224-8558 (JP); HIRUMA, Takuya, Yokohama-shi Kanagawa 224-8558 (JP)
(74) Representative: Ter Meer Steinmeister & Partner
(86) International application number: PCT/JP2016/079010
(87) International publication number: WO 2017/057675

(56) References cited:
- EP-A1- 1 123 697
- EP-A1- 2 392 312
- JP-A- S5 849 307
- JP-A- 2000 503 677
- JP-A- 2001 058 934
- JP-A- 2007 106 714
- JP-A- 2010 195 773
- US-A- 5 783 174
- US-A- 5 827 508
- US-A1- 2004 120 908

## Description

### TECHNICAL FIELD

The present invention relates to a sunscreen cosmetic. More specifically, the present invention relates to a sunscreen cosmetic containing a dibenzoylmethane derivative, which is an ultraviolet ray absorbing agent, and a ultraviolet ray scattering agent that has been subjected to a specific surface treatment, whereby the sunscreen cosmetic achieves high ultraviolet ray protection performance across a wide wavelength range from UVA to UVB, while also having excellent stability.

### BACKGROUND ART

Protecting the skin from damage due to ultraviolet rays is an important problem in skin care and body care, and various UV-care cosmetics for minimizing the harmful effects of ultraviolet rays on the skin have been developed. Sunscreen cosmetics, which are a type of UV-care cosmetic, are cosmetics that are intended to protect the skin from damage due to ultraviolet rays by covering the skin with a coating that contains an ultraviolet ray absorbing agent or an ultraviolet ray scattering agent, thereby absorbing or scattering UVA and UVB rays, and limiting the amount of ultraviolet rays that reach the skin (Non-Patent Document 1).

Dibenzoylmethane derivatives are commonly used in sunscreen cosmetics as organic ultraviolet ray absorbing agents having an absorption peak in the UVA range, but have problems such as having reduced photoprotection performance due to photodecomposition and crystal precipitation or discoloration (yellowing) due to interactions with other coexisting ingredients, particularly metal ions.

In Patent Document 1, discoloration (yellowing) or reduction of photoprotection capacity in a composition containing a dibenzoylmethane derivative and titanium oxide is suppressed by coating the surface of titanium oxide with silicone (a siloxane or silane derivative) (paragraphs [0009] to [0012]).

Patent Document 2 discloses the surface treatment of zinc oxide particles in order to prevent deterioration due to reactions between dibenzoylmethane and zinc oxide, and describes, as examples of the surface-treating substance, silicones, fatty acids, proteins, peptides, amino acids, N-acyl amino acids, monoglycerides, diglycerides, triglycerides, mineral oils, silica, phospholipids, sterols, hydrocarbons, polyacrylates, alumina and mixtures thereof (page 13).

While metal oxide powders such as titanium oxide and zinc oxide are blended into sunscreen cosmetics as ultraviolet ray scattering agents, they are sometimes surface-hydrophobized for the purpose of increasing their dispersibility or transparency in oil. For example, Patent Document 3 describes that when fine particles of titanium oxide surface-treated with a metal soap are used, the transparency is improved, but the ultraviolet ray screening effect is lowered (page 3).

However, since some common surface-treating substances (e.g., alumina) such as those that are named in the aforementioned Patent Document 1 or 2 contain a metal, the possibility that a powder surface-treated with these substances could undergo discoloration or crystal precipitation when coexisting with dibenzoylmethane derivatives cannot be denied. On the other hand, when blending in silica-treated titanium oxide, there was a tendency for the whiteness to be unnaturally noticeable upon application to the skin.

### RELATED ART DOCUMENTS

### PATENT DOCUMENTS

Patent Document 1: JP H9-2929 A
Patent Document 2: JP 2000-503677 A
Patent Document 3: JP S58-49307 A

### NON-PATENT DOCUMENTS

Non-Patent Document 1: Shin-keshohingaku, 2nd edition, edited by Takeo Mitsui, 2001, published by Nanzando, pp. 497-504.

### SUMMARY OF THE INVENTION

### PROBLEMS TO BE SOLVED BY THE INVENTION

Thus, the problem addressed by the present invention is to provide a sunscreen cosmetic that solves the problem of instability of dibenzoylmethane derivatives, which are UVA absorbing agents, that can achieve excellent ultraviolet ray protection performance across a wide wavelength range from UVA to UVB, that has excellent light resistance, is stable over time, does not undergo discoloration or crystal precipitation, and does not become unnaturally white.

### MEANS FOR SOLVING THE PROBLEMS

As a result of performing diligent research towards solving the above-mentioned problem, the present inventors discovered that a stable sunscreen cosmetic that does not undergo discoloration or crystal precipitation even when coexisting with a dibenzoylmethane derivative can be obtained by subjecting the surface of a metal oxide, which is blended as an ultraviolet ray scattering agent, to a treatment by a metal soap consisting of a higher fatty acid and an alkaline earth metal, or to a combination treatment by a higher fatty acid and an alkaline earth metal hydroxide, thereby completing the present invention.

In other words, the present invention provides a sunscreen cosmetic according to claim 1 comprising:
(a) a dibenzoylmethane derivative; and
(b) a powder that is surface-hydrophobized by treatment with a metal soap consisting of a higher fatty acid and an alkaline earth metal, or by combination treatment with a higher fatty acid and an alkaline earth metal hydroxide.

### EFFECTS OF THE INVENTION

By co-blending a powder (ultraviolet ray scattering agent) that has been subjected to a specific surface treatment with a dibenzoylmethane derivative which is known to have the properties of undergoing deterioration or decomposition, the present invention is able to provide a sunscreen cosmetic for which the ultraviolet ray screening performance will not decrease during storage, and for which problems in usage or in appearance, such as crystal precipitation or discoloration, will not occur.

### MODES FOR CARRYING OUT THE INVENTION

The sunscreen cosmetic of the present invention comprises, as essential ingredients, (a) a dibenzoylmethane derivative; and (b) a powder that is surface-hydrophobized by treatment with a metal soap consisting of a higher fatty acid and an alkaline earth metal, or by combination treatment with a higher fatty acid and an alkaline earth metal hydroxide.

### (a) Dibenzoylmethane derivative

Dibenzoylmethane derivatives (ingredient a) are widely used in cosmetics and the like as ultraviolet ray absorbing agents having maximum absorption wavelengths in the UVA range.

Dibenzoylmethane derivatives which are used in the present invention include 2-methyl dibenzoylmethane, 4-methyl dibenzoylmethane, 4-isopropyl dibenzoylmethane, 4-tert-butyl dibenzoylmethane, 2,4-dimethyl dibenzoylmethane, 2,5-dimethyl dibenzoylmethane, 4,4'-diisopropyl dibenzoylmethane, 4-methoxy-4'-tert-butyl dibenzoylmethane, 2-methyl-5-isopropyl-4'-methoxy dibenzoylmethane, 2-methyl-5-tert-butyl-4' -methoxy dibenzoylmethane, 2,4-dimethyl-4' -methoxy dibenzoylmethane, 2,6-dimethyl-4-tert-butyl-4'-methoxy dibenzoylmethane or the like.

Among the aforementioned dibenzoylmethane derivatives, it is particularly preferable to use 4-methoxy-4'-tert-butyl dibenzoylmethane (label name: t-butyl methoxy benzoylmethane).

The dibenzoylmethane derivative may be a commercially available product, an example of which is the 4-methoxy-4'-tert-butyl dibenzoylmethane that is commercially available from DSM Nutrition Japan under the name "Parsol 1789".

The blended amount of the dibenzoylmethane derivative in the sunscreen cosmetic of the present invention is preferably 0.5 to 4.0 mass%, more preferably 1.0 to 3.0 mass%. If the blended amount is less than 0.5 mass%, then sufficient ultraviolet ray protection effects cannot be obtained, and even if more than 4.0 mass% is blended in, there is no further improvement in the ultraviolet ray protection effects, and there may be problems in the properties when used, such as stickiness.

### (b) Powder (ultraviolet ray scattering agent)

The powder that is blended into the sunscreen cosmetic of the present invention is a powder (also known as an "ultraviolet ray scattering agent") that physically screens ultraviolet rays by reflection or scattering, wherein the powder has been subjected to a specific surface hydrophobization treatment.

The specific hydrophobization treatment in the present invention refers to (1) treatment with a metal soap consisting of a higher fatty acid and an alkaline earth metal, or (2) combination treatment with a higher fatty acid and an alkaline earth metal hydroxide. The higher fatty acid used here is a straight or branched carboxylic acid having 8 to 24 carbon atoms (preferably 12 to 18 carbon atoms). Specific examples include stearic acid, isostearic acid, myristic acid, lauric acid and the like, and isostearic acid is particularly preferred.

The alkaline earth metal is calcium or magnesium, and magnesium is particularly preferred.

It is thought that, when a powder surface is subjected to a combination treatment with a higher fatty acid and an alkaline earth metal hydroxide, a layer of a metal soap consisting of an alkaline earth metal salt and the higher fatty acid is formed on the powder surface. In other words, it is inferred that the surface treatment layers formed on the powder surface by the aforementioned treatments (1) or (2) have the same composition.

The hydrophobization method is not particularly limited, and it is possible to use wet methods using solvents, vapor-phase deposition methods, mechanochemical methods or the like.

The powder (nucleus) that is subjected to the specific hydrophobization treatment is not particularly limited as long as it is a powder that is used as an ultraviolet ray scattering agent in the field of cosmetics. Specific examples include one or more types chosen from among titanium oxide, zinc oxide, barium sulfate, iron oxide, talc, mica, sericite, kaolin, titanated mica, Prussian blue, chromium oxide, chromium hydroxide, silica, cerium oxide and the like. In particular, it is preferable, in terms of optical properties, to use a powder having a refractive index of at least 1.5.

The powder (nucleus) is not particularly limited as long as it is a powder that has been conventionally blended into cosmetics and the like. For example, a powder having an average primary particle size of 100 nm or less, preferably 50 nm or less, and more preferably 30 nm or less, may be favorably used. The lower limit of the average primary particle size is not particularly limited, but powders become more expensive as the particle sizes become smaller, so particles sizes of at least 5 nm are acceptable, and particle sizes of at least 10 nm are preferred when taking economic factors into consideration. In the present specification, average primary particle size refers to the size of primary particles in a powder, as measured by a commonly used method, and specifically may be a value that is determined as the arithmetic mean of the lengths of the long axes and the lengths of the short axes of the particles based on transmission electron microscope photographs.

Additionally, the forms of the particles in the powder are not particularly limited, and they may be spherical, elliptical, crushed or the like, and the particles may be in the form of primary particles, or may form aggregated secondary clusters.

Hereinbelow, the aforementioned powder that has been subjected to a specific treatment (ingredient b) may be referred to simply as the "ultraviolet ray scattering agent".

The blended amount of the ultraviolet ray scattering agent (ingredient b) in the sunscreen cosmetic of the present invention is preferably 1 to 15 mass%, more preferably 2 to 8 mass%. If the blended amount is less than 1 mass%, then sufficient ultraviolet ray protection effects cannot be obtained, and if the blended amount exceeds 15 mass%, there is a tendency for the properties when used to be lowered.

In the sunscreen cosmetic of the present invention, the blended amount ratio between (a) the dibenzoylmethane derivative and (b) the ultraviolet ray scattering agent should preferably be adjusted so that (a) : (b) = 1 : 24 to 3 : 1, more preferably 1 : 10 to 2 : 1, and even more preferably 1 : 5 to 1 : 1. By setting the blended amount ratio to be in the aforementioned ranges, ultraviolet ray protection effects are effectively obtained spanning across a wavelength range from UVA to UVB.

Dibenzoylmethane derivatives, particularly 4-methoxy-4'-tert-butyl dibenzoylmethane, have conventionally been generally used in cosmetics as organic UVA-range ultraviolet ray absorbing agents, and powders such as titanium oxide are known as ultraviolet ray scattering agents, and various types of surface-hydrophobized powders have been used. However, the type of metal soap that is most commonly used in the surface treatment of powders is metal soap containing aluminum, which is a trivalent metal, and no examples combining a dibenzoylmethane derivative with a powder that is surface-treated with a metal soap containing an alkaline earth metal such as magnesium is known.

In the present invention, it was discovered for the first time that, by choosing a metal soap from among generally known surface treatment agents, and further choosing, from among metal soaps, a metal soap containing an alkaline earth metal, preferably calcium or magnesium, the stability can be maintained without the occurrence of crystal precipitation, even when the metal soap is blended together with a dibenzoylmethane derivative.

Therefore, the present invention provides a method for stabilizing a composition comprising a dibenzoylmethane derivative, the method comprising a step of blending in a powder that is surface-hydrophobized by treatment with a metal soap consisting of a higher fatty acid and an alkaline earth metal, or by combination treatment with a higher fatty acid and an alkaline earth metal hydroxide.

The sunscreen cosmetic of the present invention may contain, in addition to (a) the dibenzoylmethane derivative and (b) the ultraviolet ray scattering agent, other optional ingredients that can normally be blended into sunscreen cosmetics, within a range not interfering with the effects of the present invention.

While the other optional ingredients are not particularly limited, the ultraviolet ray protection performance in the UVA and/or the UVB range can be further improved, for example, by further adding ultraviolet ray absorbing agents other than the dibenzoylmethane derivative.

The ultraviolet ray absorbing agents other than the dibenzoylmethane derivative are not particularly limited and may be chosen from among those that are normally used in cosmetics. Examples include one or more substances chosen from among para-aminobenzoic acid derivatives, salicylic acid derivatives, cinnamic acid derivatives, β,β-diphenyl acrylate derivatives, benzophenone derivatives, benzylidene camphor derivatives, phenyl benzimidazole derivatives, triazine derivatives, phenyl benzotriazole derivatives, anthranil derivatives, imidazoline derivatives, benzalmalonate derivatives, 4,4-diaryl butadiene derivatives and the like.

Other optional ingredients include, but are not limited to, water-soluble polymers, oil-soluble polymers, waxes, alcohols, hydrocarbon oils, fatty acids, higher alcohols, fatty acid esters, silicone oils, surfactants, powder components other than ultraviolet ray scattering agents, pharmaceutical agents and the like.

Examples of water-soluble polymers include homopolymers or copolymers of 2-acrylamido-2-methylpropane sulfonic acid (hereinafter abbreviated to "AMPS"). The copolymers are copolymers comprising comonomers such as vinyl pyrrolidone, acrylic acid amides, sodium acrylate and hydroxyethyl acrylate. In other words, examples include AMPS homopolymers, vinyl pyrrolidone/AMPS copolymers, dimethyl acrylamide/AMPS copolymers, acrylic acid amide/AMPS copolymers, sodium acrylate/AMPS copolymers and the like.

Further examples include carboxyvinyl polymers, ammonium polyacrylate, sodium polyacrylates, sodium acrylate/alkyl acrylate/sodium methacrylate/alkyl methacrylate copolymers, carrageenan, pectin, mannan, curdlan, chondroitin sulfate, starch, glycogen, gum arabic, sodium hyaluronate, tragacanth gum, xanthan gum, mucoitin sulfate, hydroxyethyl guar gum, carboxymethyl guar gum, guar gum, dextran, keratosulfate, locust bean gum, succcinoglucan, chitin, chitosan, carboxymethyl chitin, agar and the like.

Examples of the oil-soluble polymer include trimethylsiloxysilicate, alkyl-modified silicone, polyamide-modified silicone, dimethicone cross-polymers, (dimethicone/vinyl dimethicone) cross-polymers, poylmethylsilsesquioxane and the like.

Examples of waxes include beeswax, candelilla wax, carnauba wax, lanolin, liquid lanolin, jojoba wax and the like.

Examples of alcohols include lower alcohols such as ethanol and isopropanol, higher alcohols such as isostearyl alcohol, octyl dodecanol and hexyl decanol, and polyhydric alcohols such as ethylene glycol, propylene glycol, 1,3-butylene glycol, dipropylene glycol and polybutylene glycol.

Examples of hydrocarbon oils include liquid paraffin, ozokerite, squalane, pristane, paraffin, ceresin, squalene, vaselin, microcrystalline wax, polyethylene wax, Fischer-Tropsch waxes and the like.

Examples of fatty acids include lauric acid, myristic acid, palmitic acid, stearic acid, behenic acid, arachidonic acid and the like.

Examples of higher alcohols include lauryl alcohol, myristyl alcohol, cetyl alcohol, stearyl alcohol, behenyl alcohol, arachyl alcohol, batyl alcohol, chimyl alcohol, carnaubyl alcohol, ceryl alcohol, corianyl alcohol, myricyl alcohol, lacceryl alcohol, elaidyl alcohol, isostearyl glyceryl ether, octyl alcohol, triacontyl alcohol, selachyl alcohol, cetostearyl alcohol, oleyl alcohol, lanolin alcohol, hydrogenated lanolin alcohol, hexyl decanol, octyl decanol and the like.

Examples of fatty acid esters include myristyl myristate, cetyl palmitate, cholesteryl stearate, 2-octyldodecyl beeswax fatty acid and the like.

Examples of silicone oils include methyl polysiloxane, octamethylsiloxane, decamethyltetrasiloxane, methyl hydrogen polysiloxane, methyl phenyl polysiloxane, hexamethylcyclotrisiloxane, octamethylcyclotetrasiloxane, decamethylcyclopentasiloxane and the like. Preferable examples include octamethylcyclotetrasiloxane, decamethylcyclopentasiloxane and the like.

Examples of surfactants include anionic, cationic, non-ionic or amphoteric surfactants, including silicone-based or hydrocarbon-based surfactants.

Examples of powder ingredients other than the ultraviolet ray scattering agent (ingredient b) include nylon or acrylic polymer spherical powders, silica powders, silicone powders, metal oxide powders that have been surface-treated with surface treating agents not containing metals, and the like. However, metal oxide powders that have not been surface-treated, such as, for example, zine oxide powders, should preferably not be blended because there is a possibility that they will interfere with the stability of the dibenzoylmethane derivative.

Examples of pharmaceutical agents include L-ascorbic acid and derivative salts thereof, glycyrrhizic acid and derivatives thereof such as dipotassium glycyrrhizate and monoammonium glycyrrhizate, glycyrrhetinic acid and derivatives thereof such as stearyl glycyrrhetinate, allantoin, tranexamic acid and derivative salts thereof, alkoxysalicylic acid and derivative salts thereof, glutathione and derivative salts thereof, allantoin, azulene and the like.

The sunscreen cosmetic of the present invention may be provided in the form of an oil-in-water emulsified cosmetic, a water-in-oil emulsified cosmetic or an oil-based cosmetic. Specific formats include formats such as sunscreen lotions and sunscreen creams, which may be manufactured using conventional methods that are appropriate for each format.

### EXAMPLES

Herebelow, the present invention will be described in further detail by giving examples, but the present invention is not to be construed as being limited thereto. Where not specifically noted, blended amounts are indicated in mass% with respect to the system in which the ingredient is contained.

Samples of water-in-oil emulsified sunscreen cosmetics were prepared with the formulations indicated in Table 1 below. Next, the samples of the formulated examples were evaluated as to (1) crystal precipitation, (2) whiteness when applied, and (3) dispersibility in oil, as indicated below. The evaluation results are also shown in Table 1.

### (1) Crystal precipitation

Samples were stored for one month in a temperature cycle from -10 °C to 20 °C, then observed through a polarizing microscope for the presence or absence of crystal precipitation.
A: No crystal precipitation observed.
B: Crystal precipitation observed.

### (2) Whiteness when applied

Female panelists (ten) applied samples of each of the examples and comparative examples, and evaluated them for whiteness on the basis of the following evaluation criteria.

### (Evaluation)

A: 4 or fewer responded that the whiteness after application was unacceptable.
B: 5 or more responded that the whiteness after application was unacceptable.

### (3) Dispersibility in oil (presence or absence of powder aggregation or the like)

Visual observations were made as to the presence or absence of small aggregated clusters (powder clumps) or color variations caused by powder aggregation when a sample of each example was applied to a black plate.

### (Evaluation)

A: Absolutely no small aggregated clusters of powder or powder color variations caused by aggregation were observed when the powder was applied to the plate.
B: Very small aggregated clusters of powder and slight powder color variations caused by aggregation were observed when the powder was applied to the plate.
C: Small aggregated clusters of powder and powder color variations caused by aggregation were observed when the powder was applied to the plate.

**[Table 1]**

| | | **Example** | | **Comparative Example** | | | | |
|---|---|---|---|---|---|---|---|---|
| | | **1** | **2** | **1** | **2** | **3** | **4** | **5** |
| | Ion-exchanged water | 22 | 22 | 22 | 22 | 22 | 22 | 22 |
| | PEG-9 polydimethylsiloxyethyl dimethicone | 3 | 3 | 3 | 3 | 3 | 3 | 3 |
| | Light isoparaffin | 25 | 25 | 25 | 25 | 25 | 25 | 25 |
| | Glyceryl tri-2-ethylhexanoate | 30 | 30 | 30 | 30 | 30 | 30 | 30 |
| Absorbing agent | 2-ethylhexyl paramethoxycinnamate | 10 | 10 | 10 | 10 | 10 | 10 | 10 |
| | 4-tert-butyl-4' methoxy dibenzoylmethane | 3 | 3 | 3 | 3 | 3 | 3 | 3 |
| Scattering agent | Mg-isostearate-treated fine-particle titanium oxide | 7 | - | - | - | - | - | - |
| | Mg-myristate-coated barium sulfate | - | 7 | - | - | - | - | - |
| | Hydrated-silica-coated fine-particle titanium oxide | - | - | 7 | - | - | - | - |
| | Al-stearate-treated fine-particle titanium oxide | - | - | - | 7 | - | - | - |
| | Zinc-oxide-coated crosslinked silicone/reticulated silicone block copolymer | - | - | - | - | 7 | - | - |
| | Methylpolysiloxane-coated fine-particle zinc oxide | - | - | - | - | - | 7 | - |
| | Zinc-oxide-coated talc | - | - | - | - | - | - | 7 |
| Crystal precipitation | | A | A | A | B | B | B | B |
| Whiteness when applied | | A | A | B | A | A | A | A |
| Dispersibility in oil | | A | A | B | A | A | A | A |

As is clear from the results shown in Table 1, whether the substrate (nucleus) is titanium oxide or barium sulfate, Examples 1 and 2, the surfaces of which were hydrophobized (coated) with metal soaps containing alkaline earth metals (magnesium), did not undergo crystal precipitation, did not cause a sensation of unnatural whiteness, and had good oil dispersibility for the powders (ultraviolet ray scattering agents), even when coexisting with dibenzoylmethane derivatives.

Even when the substrate (nucleus) was titanium oxide as in Example 1, Comparative Example 1, which was surface-treated with hydrated silica, did not undergo crystal precipitation, but did have an unnatural and unacceptable whiteness when applied, and also had poor oil dispersibility for the powder. In Comparative Example 2, in which titanium oxide was surface-treated with aluminum stearate, the whiteness when applied and the oil dispersibility were acceptable, but crystal precipitation occurred. In Comparative Examples 3 and 5, for which the surfaces were composed of zinc oxide, crystal precipitation occurred whether the substrate (nucleus) was a silicone resin or a magnesium-containing compound (talc), and crystal precipitation was similarly observed in Comparative Example 4, which was surface-treated with zinc oxide.

From the above, it is clear that the effect of being able to suppress crystal precipitation with benzoylmethane derivatives by performing a surface treatment with a metal soap containing an alkaline earth metal in the present invention can be achieved irrespective of the type of substrate (nucleus) used for the powder, but the effects of the present invention cannot be obtained when the metal in the surface treatment agent is replaced with a silicone, a metal oxide or a metal soap containing a trivalent metal (aluminum).

In order to confirm the direct impact that various types of metal ions have on dibenzoylmethane derivatives, the following tests were performed.

Water-in-oil emulsified compositions (samples) were prepared using the formulations shown in the following Table 2. The blended amounts of the metal chlorides were determined so as to equalize the number of moles of metal ions contained in the samples.

First, the oil phase part (light isoparaffin, decamethylcyclopentasiloxane, glyceryl tri-2-ethylhexanoate, PEG-9 polydimethylsiloxyethyl dimethicone, 2-ethylhexyl para-methoxycinnamate and 4-tert-butyl-4'-methoxy dibenzoylmethane) was added to a beaker and stirred/mixed, and in a separate container, the metal chlorides were added/dissolved in ion-exchanged water to obtain the water phase part. Next, the water phase part was added to the oil phase part and the resulting mixture was emulsified with a homogenizer.

The obtained samples were measured for the presence or absence of tone changes (discoloration) as indicated below.

### <Measurement Method>

Each sample was stored for 1 month at 50 °C, then visually observed for the presence or absence of discoloration.

### <Measurement Results>

A: Discoloration not observed.
B: Discoloration observed.

**[Table 2]**

| | Example 3 | Example 4 | Comparative Example 6 | Comparative Example 7 |
|---|---|---|---|---|
| Ion-exchanged water | 17 | 18.9 | 15.7 | 19.3 |
| PEG-9 polydimethylsiloxyethyl dimethicone | 3 | 3 | 3 | 3 |
| Light isoparaffin | 20 | 20 | 20 | 20 |
| Decamethylcyclopentasiloxane | 5 | 5 | 5 | 5 |
| Glyceryl tri-2-ethylhexanoate | 35 | 35 | 35 | 35 |
| 2-Ethylhexyl para-methoxycinnamate | 10 | 10 | 10 | 10 |
| 4-tert-butyl-4' -methoxy dibenzoylmethane | 3 | 3 | 3 | 3 |
| Magnesium chloride hexahydrate | 7 | - | - | - |
| Calcium chloride divalent salt | - | 5.1 | - | - |
| Aluminum(III) chloride hexahydrate | | - | 8.3 | - |
| Zinc chloride | - | - | - | 4.7 |
| Tone change (discoloration) | A | A | B | B |

Based on the results shown in Table 2, it was confirmed that discoloration of the dibenzoylmethane derivative (4-tert-butyl-4'-methoxy dibenzoylmethane) did not occur even when coexisting with ions of magnesium and calcium, which are alkaline earth metals, but discoloration did occur when coexisting with aluminum, which is another type of metal, or with zinc, which is a transition metal.

Formulation examples of sunscreen cosmetics according to the present invention are shown below, but the scope of the present invention is not to be construed as being limited thereto.

**Formulation 1: W/O Sunscreen**

| Blended Ingredient | Blended Amount (mass%) |
|---|---|
| Ion-exchanged water | balance |
| Ethanol | 5 |
| Glycerin | 3 |
| PEG-9 Polydimethylsiloxyethyl dimethicone | 2 |
| Light isoparaffin | 20 |
| Glyceryl tri-2-ethylhexanoate | 15 |
| Cetyl 2-ethylhexanoate | 10 |
| Decamethylcyclopentasiloxane | 15 |
| 4-tert-butyl-4'-methoxy dibenzoylmethane | 3 |
| Diethylamino hydroxybenzoyl hexyl benzoate | 1 |
| *Bupleurum scorzonerifolium* extract | 0.05 |
| Hydrolyzed conchiolin | 0.03 |
| Theanine | 0.01 |
| Magnesium-isostearate-treated fine-particle titanium oxide | 10 |
| Magnesium-myristate-coated barium sulfate | 3 |

**Formulation 2: P/O/W Sunscreen**

| Blended Ingredient | Blended Amount (mass%) |
|---|---|
| Ion-exchanged water | balance |
| Ethanol | 5 |
| Glycerin | 3 |
| 1,3-Butylene glycol | 5 |
| Polyoxyethylene hydrogenated castor oil | 1.5 |
| Octocrylene | 5 |
| 2-Ethylhexyl para-methoxycinnamate | 5 |
| Glyceryl tri-2-ethylhexanoate | 10 |
| Light isoparaffin | 10 |
| 4-tert-butyl-4'-methoxy dibenzoylmethane | 2 |
| 2,4,6-tris[4-(2-ethylhexyloxycarbonyl)anilino]-1,3,5-triazine | 2 |
| 2,4-bis-[{4-(2-ethylhexyloxy)-2-hydroxy} -phenyl]-6-(4-methoxyphenyl)-1,3,5-triazine | 0.5 |
| Magnesium-isostearate-treated fine-particle titanium oxide | 5 |
| Isostearic acid | 0.5 |
| Dimethylacrylamide/Na acryloyldimethyl taurate cross-polymer | 0.3 |
| Succinoglucan | 0.2 |
| Spherical cellulose powder | 3 |
| Tranexamic acid | 2 |
| Hydrolyzed silk | 0.01 |
| Hibiscus flower extract | 0.1 |
| Na acetylhyaluronate | 0.0001 |
| Thyme extract | 0.01 |
| Turmeric extract | 0.03 |
| EDTA-3Na·2H₂O | 0.001 |

**Formulation 3: Makeup Base**

| Blended Ingredient | Blended Amount (mass%) |
|---|---|
| Ion-exchanged water | balance |
| Dipropylene glycol | 9 |
| Polyoxyethylene hydrogenated castor oil | 1 |
| Carboxymethyl cellulose | 0.1 |
| Xanthan gum | 0.6 |
| Sorbitan sesquiisostearate | 0.35 |
| Isostearic acid | 0.5 |
| Isopropyl myristate | 2 |
| Isododecane | 12 |
| 4-tert-butyl-4'-methoxy dibenzoylmethane | 1.5 |
| Octocrylene | 5 |
| Oxybenzone | 1.5 |
| Magnesium-isostearate-treated pigment grade titanium oxide | 1 |
| Magnesium-isostearate-treated fine-particle titanium oxide | 5 |
| Spherical cellulose powder | 2 |
| Porous silica | 1 |
| Potassium 4-methoxysalicylate | 1 |
| *Angelica acutiloba* root extract | 0.001 |
| PEG/PPG-14/7 dimethyl ether | 1 |
| PEG-12 dimethicone | 0.01 |
| Polyquaternium-51 | 0.04 |

**Formulation 4: W/O Sunscreen**

| Blended Ingredient | Blended Amount (mass%) |
|---|---|
| Ion-exchanged water | balance |
| Ethanol | 2 |
| Glycerin | 3 |
| Dipropylene glycol | 5 |
| PEG-9 Polydimethylsiloxyethyl dimethicone | 2 |
| Light isoparaffin | 20 |
| Glyceryl tri-2-ethylhexanoate | 15 |
| Dimethicone | 5 |
| 2-Ethylhexyl succinate | 5 |
| Decamethylcyclopentasiloxane | 15 |
| 4-tert-butyl-4'-methoxy dibenzoylmethane | 3 |
| Phenylbenzimidazole sulfonic acid | 0.5 |
| Triethanolamine | 0.2 |
| Calcium-myristate-coated talc | 5 |
| Dipotassium glycyrrhizinate | 0.001 |
| Vitamin C ethyl | 0.01 |
| Trehalose | 0.5 |

## Claims

1. A sunscreen cosmetic comprising:
(a) a dibenzoylmethane derivative consisting of one or more selected from 2-methyl dibenzoylmethane, 4-methyl dibenzoylmethane, 4-isopropyl dibenzoylmethane, 4-tert-butyl dibenzoylmethane, 2,4-dimethyl dibenzoylmethane, 2,5-dimethyl dibenzoylmethane, 4,4'-diisopropyl dibenzoylmethane, 4-methoxy-4'-tert-butyl dibenzoylmethane, 2-methyl-5 -isopropyl-4' -methoxy dibenzoylmethane, 2-methyl-5-tert-butyl-4' -methoxy dibenzoylmethane, 2,4-dimethyl-4'-methoxy dibenzoylmethane, and 2,6-dimethyl-4-tert-butyl-4'-methoxy dibenzoylmethane; and
(b) a powder that is surface-hydrophobized by treatment with a metal soap consisting of a higher fatty acid which is a straight or branched carboxylic acid having 8 to 24 carbon atoms and calcium or magnesium, or by combination treatment with a higher fatty acid which is a straight or branched carboxylic acid having 8 to 24 carbon atoms and calcium hydroxide or magnesium hydroxide.

2. The cosmetic as in claim 1, wherein the higher fatty acid is a straight or branched carboxylic acid having 12 to 18 carbon atoms.

3. The cosmetic as in any one of claims 1 or 2, wherein the blended amount of the (a) dibenzoylmethane derivative is 0.5 to 4.0 mass%, and the blended amount of the (b) powder that is surface-hydrophobized is 1.0 to 15.0 mass%.

4. The cosmetic as in any one of claims 1 to 3, wherein the blended amount ratio between the blended amount of the (a) dibenzoylmethane derivative and the blended amount of the (b) powder that is surface-hydrophobized is within the range, (a) : (b) = 1 : 24 to 3 : 1.

5. A method for stabilizing a composition comprising a dibenzoylmethane derivative consisting of one or more selected from 2-methyl dibenzoylmethane, 4-methyl dibenzoylmethane, 4-isopropyl dibenzoylmethane, 4-tert-butyl dibenzoylmethane, 2,4-dimethyl dibenzoylmethane, 2,5-dimethyl dibenzoylmethane, 4,4'-diisopropyl dibenzoylmethane, 4-methoxy-4'-tert-butyl dibenzoylmethane, 2-methyl-5-isopropyl-4'-methoxy dibenzoylmethane, 2-methyl-5-tert-butyl-4'-methoxy dibenzoylmethane, 2,4-dimethyl-4'-methoxy dibenzoylmethane, and 2,6-dimethyl-4-tert-butyl-4'-methoxy dibenzoylmethane, the method comprising a step of blending in a powder that is surface-hydrophobized by treatment with a metal soap consisting of a higher fatty acid which is a straight or branched carboxylic acid having 8 to 24 carbon atoms and an calcium or magnesium, or by combination treatment with a higher fatty acid which is a straight or branched carboxylic acid having 8 to 24 carbon atoms and calcium hydroxide or magnesium hydroxide.

## Patentansprüche

1. Sonnenschutz-Kosmetikum, umfassend:
(a) ein Dibenzoylmethanderivat, bestehend aus einem oder mehreren, gewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisoprpoyldibenzoylmethan, 4-Methoxy-4'-tert-butyldibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, und 2,6-Dimethyl-4-tert-butyl-4'-methoxydibenzoylmethan; und
(b) ein Pulver, das oberflächen-hydrophobisiert ist durch Behandlung mit einer Metallseife, bestehend aus einer höheren Fettsäure, die eine gerade oder verzweigte Carbonsäure mit 8 bis 24 Kohlenstoffatomen ist, und Calcium oder Magnesium, oder durch Kombinationsbehandlung mit einer höheren Fettsäure, die eine gerade oder verzweigte Carbonsäure mit 8 bis 24 Kohlenstoffatomen ist, und Calciumhydroxid oder Magnesiumhydroxid.

2. Kosmetikum nach Anspruch 1, wobei die höhere Fettsäure eine gerade oder verzweigte Carbonsäure mit 12 bis 18 Kohlenstoffatomen ist.

3. Kosmetikum nach irgendeinem der Ansprüche 1 oder 2, wobei die Mischungsmenge des (a) Dibenzoylmethanderivats 0,5 bis 4,0 Masse-% beträgt, und die Mischungsmenge des (b) Pulvers, das oberflächen-hydrophobisiert ist, 1,0 bis 15,0 Masse-% beträgt.

4. Kosmetikum nach irgendeinem der Ansprüche 1 bis 3, wobei das Mischungsmengenverhältnis zwischen der Mischungsmenge des (a) Dibenzoylmethanderivats und der Mischungsmenge des (b) Pulvers, das oberflächen-hydrophobisiert ist, innerhalb des Bereichs (a) : (b) = 1 : 24 bis 3 : 1 liegt.

5. Verfahren zum Stabilisieren einer Zusammensetzung, umfassend ein Dibenzoylmethanderivat, bestehend aus einem oder mehreren, gewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisoprpoyldibenzoylmethan, 4-Methoxy-4'-tert-butyldibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, und 2,6-Dimethyl-4-tert-butyl-4'-methoxydibenzoylmethan, das Verfahren umfassend einen Schritt des Einmischens eines Pulvers, das oberflächen-hydrophobisiert ist durch Behandlung mit einer Metallseife, bestehend aus einer höheren Fettsäure, die eine gerade oder verzweigte Carbonsäure mit 8 bis 24 Kohlenstoffatomen ist, und Calcium oder Magnesium, oder durch Kombinationsbehandlung mit einer höheren Fettsäure, die eine gerade oder verzweigte Carbonsäure mit 8 bis 24 Kohlenstoffatomen ist, und Calciumhydroxid oder Magnesiumhydroxid.

## Revendications

1. Cosmétique d'écran solaire comprenant :
(a) un dérivé de dibenzoylméthane constitué d'un ou de plusieurs dérivés choisis parmi le 2-méthyldibenzoylméthane, le 4-méthyldibenzoylméthane, le 4-isopropyldibenzoylméthane, le 4-tert-butyldibenzoylméthane, le 2,4-diméthyldibenzoylméthane, le 2,5-diméthyldibenzoylméthane, le 4,4'-diisopropyldibenzoylméthane, le 4-méthoxy-4'-tert-butyldibenzoylméthane, le 2-méthyl-5-isopropyl-4'-méthoxydibenzoylméthane, le 2-méthyl-5-tert-butyl-4'-méthoxydibenzoylméthane, le 2,4-diméthyl-4'-méthoxydibenzoylméthane et le 2,6-diméthyl-4-tert-butyl-4'-méthoxydibenzoylméthane ; et
(b) une poudre qui est hydrophobisée en surface par traitement avec un savon métallique constitué d'un acide gras supérieur qui est un acide carboxylique linéaire ou ramifié ayant 8 à 24 atomes de carbone et du calcium ou du magnésium, ou par traitement combiné avec un acide gras supérieur qui est un acide carboxylique linéaire ou ramifié ayant 8 à 24 atomes de carbone et de l'hydroxyde de calcium ou de l'hydroxyde de magnésium.

2. Cosmétique selon la revendication 1, l'acide gras supérieur étant un acide carboxylique linéaire ou ramifié ayant 12 à 18 atomes de carbone.

3. Cosmétique selon l'une quelconque des revendications 1 ou 2, la quantité mélangée du (a) dérivé de dibenzoylméthane étant de 0,5 à 4,0 % en masse, et la quantité mélangée de (b) la poudre qui est hydrophobisée en surface étant de 1,0 à 15,0 % en masse.

4. Cosmétique selon l'une quelconque des revendications 1 à 3, le rapport de quantité mélangée entre la quantité mélangée du (a) dérivé de dibenzoylméthane et la quantité mélangée de (b) la poudre qui est hydrophobisée en surface se trouvant à l'intérieur de la plage, (a) : (b) = 1 : 24 à 3 : 1.

5. Procédé de stabilisation d'une composition comprenant un dérivé de dibenzoylméthane constitué d'un ou de plusieurs dérivés choisis parmi le 2-méthyldibenzoylméthane, le 4-méthyldibenzoylméthane, le 4-isopropyldibenzoylméthane, le 4-tert-butyldibenzoylméthane, le 2,4-diméthyldibenzoylméthane, le 2,5-diméthyldibenzoylméthane, le 4,4'-diisopropyldibenzoylméthane, le 4-méthoxy-4'-tert-butyldibenzoylméthane, le 2-méthyl-5-isopropyl-4'-méthoxydibenzoylméthane, le 2-méthyl-5-tert-butyl-4'-méthoxydibenzoylméthane, le 2,4-diméthyl-4'-méthoxydibenzoylméthane et le 2,6-diméthyl-4-tert-butyl-4'-méthoxydibenzoylméthane, le procédé comprenant une étape de mélange dans une poudre qui est hydrophobisée en surface par traitement avec un savon métallique constitué d'un acide gras supérieur qui est un acide carboxylique linéaire ou ramifié ayant 8 à 24 atomes de carbone et du calcium ou du magnésium, ou par traitement combiné avec un acide gras supérieur qui est un acide carboxylique linéaire ou ramifié ayant 8 à 24 atomes de carbone et de l'hydroxyde de calcium ou de l'hydroxyde de magnésium.
